# EUROPEAN PATENT SPECIFICATION

(11) **EP 2 316 384 B1**
(45) Date of publication and mention of the grant of the patent: **03.04.2013**
(21) Application number: 10188962.4
(22) Date of filing: 26.10.2010
(51) Int. Cl.: A61F 2/38

(54) **Prosthesis with modular extensions**
Prothese mit modularen Erweiterungen
Prothèse dotée d'extensions modulaires

(30) Priority: 30.10.2009 US 256527 P; 17.11.2009 US 620034
(43) Date of publication of application: 04.05.2011
(73) Proprietor: DePuy Products, Inc., Warsaw, IN 46581 (US)
(72) Inventor: Deffenbaugh, Daren L, Warsaw, IN 46590 (US); Zannis, Anthony D, Fort Wayne, IN 46845-9026 (US)
(74) Representative: Belcher, Simon James

(56) References cited:
- WO-A2-2009/128943
- FR-A1- 2 780 636
- US-A- 5 755 800
- US-A1- 2004 015 170

## Description

The present invention relates generally to an implantable knee joint.

During the lifetime of a patient, it may be necessary to perform a joint replacement procedure on the patient as a result of, for example, disease or trauma. The joint replacement procedure may involve the use of a prosthesis that is implanted into one or more of the patient's bones. In the case of a knee replacement procedure, a tibial tray is implanted into the patient's tibia. A bearing is then secured to the tibial tray. The condyle surfaces of a replacement femoral component bear against the tibial bearing.

One type of knee prosthesis is a fixed-bearing knee prosthesis. As its name suggests, the bearing of a fixed-bearing knee prosthesis does not move relative to the tibial tray. Fixed-bearing designs are commonly used when the condition of the patient's soft tissue (i.e., knee ligaments) does not allow for the use of a knee prosthesis having a mobile bearing.

In contrast, in a mobile-bearing type of knee prosthesis, the bearing can move relative to the tibial tray. Mobile-bearing knee prostheses include so-called "rotating platform" knee prostheses, in which the bearing can rotate about a longitudinal axis on the tibial tray.

Tibial trays are commonly made of a biocompatible metal, such as a cobalt chromium alloy or a titanium alloy.

For both fixed and mobile-bearing knee prostheses, the tibial trays may be designed to be cemented into place on the patient's tibia or alternatively may be designed for cementless fixation. Cemented fixation relies on mechanical bonds between the tibial tray and the cement as well as between the cement and the bone. Cementless implants generally have surface features that are conducive to bone ingrowth into the implant component and rely to a substantial part on this bony ingrowth for secondary fixation; primary fixation is achieved through the mechanical fit of the implant and the prepared bone.

Tibial components of both fixed and mobile-bearing and cemented and cementless knee arthroplasty systems are commonly modular components, comprising a tibial tray and a polymeric bearing carried by the tibial tray. The tibial trays commonly include features extending distally, such as pegs or stems. These extensions penetrate below the surface of the tibial plateau and stabilize the tibial tray component against movement. In cementless tibial implants, the outer surfaces of these extensions are typically porous to allow for bone ingrowth. For example, in the Zimmer Trabecular Metal Monoblock tibial trays, pegs with flat distal surfaces and hexagonal axial surfaces are formed completely of a porous metal. In such trays, bone ingrowth is likely to occur along all surfaces of the pegs, including the distal surfaces.

Femoral components of such knee prosthesis systems are also designed for either cemented or cementless fixation. For cemented fixation, the femoral component typically includes recesses or cement pockets. For cementless fixation, the femoral component is designed for primary fixation through a press-fit, and includes porous bone-engaging surfaces suitable for bone ingrowth. Both designs may include pegs designed to extend into prepared holes in the femur for stabilization of the implant.

On occasion, the primary knee prosthesis fails. Failure can result from many causes, including wear, aseptic loosening, osteolysis, ligamentous instability, arthrofibrosis and patellofemoral complications. When the failure is debilitating, revision surgery may be necessary. In a revision, the primary knee prosthesis (or parts of it) is removed and replaced with components of a revision prosthetic system.

When the tibial or femoral implant includes extensions (such as pegs or stems) that extend into the natural bone, a revision surgery usually requires a large resection of the bone in order to dislodge the extensions from the bone. This large resection not only complicates the surgery, it also requires removal of more of the patient's natural bone than is desirable. This removal of additional bone may further compromise the bone, increase the risk of onset of bone pathologies or abnormalities, or reduce the available healthy bone for fixation of the revision implant. Moreover, the large resection usually means that a larger orthopaedic implant is necessary to fill the space and restore the joint component to its expected geometry.

This difficulty in dislodging the primary implant components from the bones is worsened by the fact that bone also grows into the extensions. Severing these connections may be problematic since not all of these areas are easily accessible without resecting large amounts of bone.

WO-A-2009/128943 discloses an artificial knee joint in which the tibial component includes an augment which provides the bone facing surface of the component. The tibial component has threaded prongs on its lower face. The augment has openings formed in it which can be aligned with the prongs so that the prongs extend through the openings when the augment is in place on the tibial component. The augment is held in place on the tibial component by means of threaded nuts which can be fitted on to the prongs, within the openings in the augment. The ends of the prongs are pointed and are exposed beyond the augment so that they penetrate the surface of the prepared tibia.

The present invention provides a knee prosthesis as defined in claim 1.

Optionally, the stud is threaded and the extension includes a threaded bore engaging the stud.

Optionally, the stud defines a Morse taper post and the extension includes a Morse taper bore engaging the stud.

Optionally, the first component includes a solid metal portion and the recess and stud are part of the solid metal portion. The first component may further include a porous metal portion that defines the bone-engaging surface of the first component.

Optionally, the porous metal portion and the extension comprise titanium metal foam and the solid metal portion comprises a titanium alloy.

Optionally, the porous metal portion, solid metal portion and extension are bonded together through sintering and the extension and the porous metal portion meet at a junction. The junction of the extension and the porous metal portion may comprise titanium metal foam.

Optionally, the joint prosthesis may include a plurality of extensions. The first component may include a plurality of spaced recesses and a plurality of spaced studs, each stud within one recess and each stud having an end. Each extension is mounted on a stud and bonded to the porous metal portion and solid metal portion through sintering. Each extension meets the porous metal portion at a junction. These junctions lie in a plane. The ends of the studs do not extend beyond the plane of the junctions.

Optionally, the second component includes a solid metal portion and the recess and stud are part of the solid metal portion.

Optionally, the second component includes a porous metal portion and the bone-engaging surface is part of the porous metal portion. The porous metal portion and the extension may comprise titanium metal foam and the solid metal portion may comprise a titanium alloy. The porous metal portion, solid metal portion and extension may be bonded together through sintering and in which the extension and the porous metal portion meet at a junction. The junction of the extension and the porous metal portion may comprise titanium metal foam.

Optionally, the joint prosthesis includes a plurality of extensions and the second component includes a plurality of spaced recesses and a plurality of spaced studs, each stud being within one recess and having an end. Each extension is mounted on a stud and bonded to the porous metal portion and solid metal portion through sintering. Each extension meets the porous metal portion at a junction. The junctions lie in a plane and the ends of the studs do not extend beyond the plane of the junctions.

Optionally, the femoral component and the tibial component include a titanium alloy and the extension comprises commercially pure titanium. More particularly, the exposed outer surface of the extension comprises porous titanium.

Optionally, the extension comprises titanium foam. The extension may have a thickness at the junction and the extension may consists of titanium foam across the entire thickness of the extension at the junction. In an exemplary embodiment, at least part of the extension has a void space of at least 65% by volume.

Optionally, the exposed outer surface of the extension at the end of the extension has a different roughness than at least a part of the exposed outer surface of the extension between the end and the junction.

Optionally, the extension is selected from the group consisting of a peg and a stem.

Optionally, the bone-engaging surface of at least one of the femoral and tibial components comprises porous metal. More particularly, the porous metal may comprise commercially pure titanium.

Optionally, the mounting surface of the tibial tray may comprise solid titanium alloy and the extension may comprise a foam of commercially pure titanium; the solid titanium alloy may extend from the mounting surface toward the bone-engaging surface and the extension may be bonded to the solid titanium alloy of the tibial tray by sintering. In this embodiment, the bone-engaging surface of the tibial tray may comprise commercially pure titanium foam bonded to the solid titanium alloy of the tibial tray by sintering. The articulating surface of the femoral component may also comprise solid titanium alloy and the extension may comprise of foam of commercially pure titanium bonded to the solid titanium alloy by sintering; in this embodiment, the solid titanium alloy extends from the articulating surface toward the bone-engaging surface and the bone-engaging surface of the femoral component may comprise commercially pure titanium foam bonded to the solid titanium alloy of the femoral component by sintering.

Optionally, the outer surface of the extension has a coefficient of static friction at the end that is less than the coefficient of static friction at a point between the end and the junction between the extension and the tibial component.

The knee prosthesis of the invention can be made by a method which includes the steps of providing a solid metal base and a porous metal extension to be assembled with the solid metal base. The solid metal base has a first surface and a second surface opposite to the first surface. The porous metal extension and the second surface of the solid metal base include complementary mounting structures for assembling the porous metal extension and the solid metal base. The method includes the step of assembling the porous metal extension and the base followed by sintering the assembly of the porous metal extension and the base to bond the porous metal extension to the base.

The method may also include the step of providing a porous metal preform having a shape different than the shape of the porous metal extension.

If such a preform is provided, the method further comprises the steps of placing the porous metal preform against the second surface of the base and sintering the preform to the base.

The porous metal extension and the porous metal base may comprise an integral component and the steps of assembling the porous metal extension and the base and placing the porous metal preform against the second surface of the base are performed simultaneously.

Alternatively, the porous metal extension and the porous metal base may comprise discrete components and the steps of assembling the porous metal extension and the base and placing the porous metal preform against the second surface of the base are performed separately.

Optionally, the porous metal extension has two ends. The mounting structure of the porous metal extension is at one end and is surrounded by porous metal. The opposite end of the porous metal extension has different surface characteristics compared to the porous metal surrounding the mounting structure.

The different surface characteristics of the opposite end of the extension may be achieved by treating the opposite end of the porous metal extension to adjust its surface characteristics.

Treatment of the opposite end of the porous metal extension may comprise machining, milling or polishing.

Alternatively, treatment of the opposite end of the porous metal extension may comprise bonding the porous metal to another material. The other material may comprise solid metal or, in the alternative, polyetheretherketone (PEEK).

A prosthesis component, which comprises a body having a bone-engaging surface engaging the bone at an interface and an extension extending deeper into the bone, can be removed from a bone by a method which comprises the step of introducing a saw blade between the bone-engaging surface of the body and the bone at the interface to separate the bone-engaging surface from the bone and sawing through the extension to separate the extension from the body. The method may further comprise the step of sawing around the extension.

The present invention addresses the need for a prosthesis with a modular implant component suitable for cementless fixation that can be removed more readily from the bone in revision surgery to conserve native bone. In addition, a method of making such a prosthesis is disclosed, as well as a surgical method for removing such a prosthesis.

Embodiments of the invention are described below by way of example with reference to the accompanying drawings, in which:
FIG. 1 is an exploded perspective view of a fixed-bearing knee prosthesis;
FIG. 2 is a bottom perspective view of the bearing of the prosthesis of FIG. 1;
FIG. 3 is a perspective view of the tibial tray of the knee prosthesis of FIG. 1;
FIG. 4 is a bottom plan view of the tibial tray of FIG. 1;
FIG. 5 is a cross sectional view of the tibial tray of FIG. 4 taken along the line 5-5 of FIG. 4, as viewed in the direction of the arrows;
FIG. 6 is a bottom plan view of an alternative embodiment of a tibial tray;
FIG. 7 is a cross sectional view of the tibial tray of FIG. 6 taken along the line 7-7 of FIG. 6, as viewed in the direction of the arrows;
FIG. 8 is a perspective view of a preform for the tibial tray platform portion of the porous metal portion of the tibial tray of FIGS. 1 to 5;
FIG. 9 is a perspective view of a set of preforms for the extensions of the porous metal portion of the tibial tray of FIGS. 1 to 5;
FIG. 10 is a cross sectional view of the proximal end of the peg preform of FIG. 9 taken along line 10-10 of FIG. 9, as viewed in the direction of the arrows;
FIG. 11 is a cross sectional view similar to FIG. 10, showing the proximal end of the peg preform mounted on the solid metal portion of the tray;
FIG. 12 is a perspective view of an alternative form of peg that may be used for the tibial tray or femoral component;
FIG. 13 is a perspective view of another alternative form of peg that may be used for the tibial tray or femoral component;
FIG. 14 is a perspective view of an alternative form of preform that may be used for the porous metal portion of the tibial tray;
FIG. 15 is a cross sectional view of the proximal end of a portion of the preform of FIG. 14, taken along line 15-15 of FIG. 14, as viewed in the direction of the arrows;
FIG. 16 is a cross sectional view of the porous metal preform of FIG. 14, taken along line 16-16 of FIG. 14, as viewed in the direction of the arrows;
FIG. 17 is a bottom plan view of the solid metal preform for the tibial tray of FIGS. 4 and 5, for use with the porous metal preforms of FIGS. 8-9;
FIG. 18 is a cross sectional view of the solid metal preform of FIG. 17, taken along line 18-18 of FIG. 17, as viewed in the direction of the arrows;
FIG. 19 is a bottom plan view of an alternative solid metal preform, for use with the porous metal preform of FIGS. 14 and 16;
FIG. 20 is a cross sectional view of the solid metal preform of FIG. 19, taken along line 20-20 of FIG. 19, as viewed in the direction of the arrows;
FIG. 21 is an enlarged partial cross sectional view of a portion of the solid metal preform of FIGS. 17 and 18
FIG. 22 is an enlarged cross sectional view of a portion of the solid metal preform of FIGS. 19-20;
FIG. 23 is a view similar to FIG. 22, showing in cross section a portion of the solid metal preform of FIGS. 19, 20 and 22 assembled with the porous metal preform of FIGS. 14 and 16;
FIG. 24 is a bottom plan view of a tibial augment;
FIG. 25 is a bottom plan view of the tibial augment of FIG. 24 assembled with a tibial tray similar to that shown in FIGS. 6 and 7;
FIG. 26 is a cross sectional view of the assembly of FIG. 25, taken along line 26-26 of FIG. 25, as viewed in the direction of the arrows;
FIG. 27 is a cross-sectional view, similar to FIGS. 5 and 7, of another tibial tray;
FIG. 28 is an enlarged cross-sectional view of one of the studs and recesses of the metal preform of FIG. 27;
FIG. 29 is a cross-sectional view similar to FIG. 28, showing the proximal end of the peg preform mounted on the stud of FIG. 28;
FIG. 30 is a cross-sectional view similar to FIGS. 5, 7 and 27, of another tibial tray; and
FIG. 31 is a cross-sectional view similar to FIGS. 5, 7, 27 and 30, of another tibial tray.

Terms representing anatomical references, such as anterior, posterior, medial, lateral, superior, inferior etc, may be used throughout this specification in relation to both the orthopaedic implants described herein and a patient's natural anatomy. Such terms have well-understood meanings in both the study of anatomy and the field of orthopaedics. Use of such anatomical reference terms in the specification is intended to be consistent with their well-understood meanings unless noted otherwise.

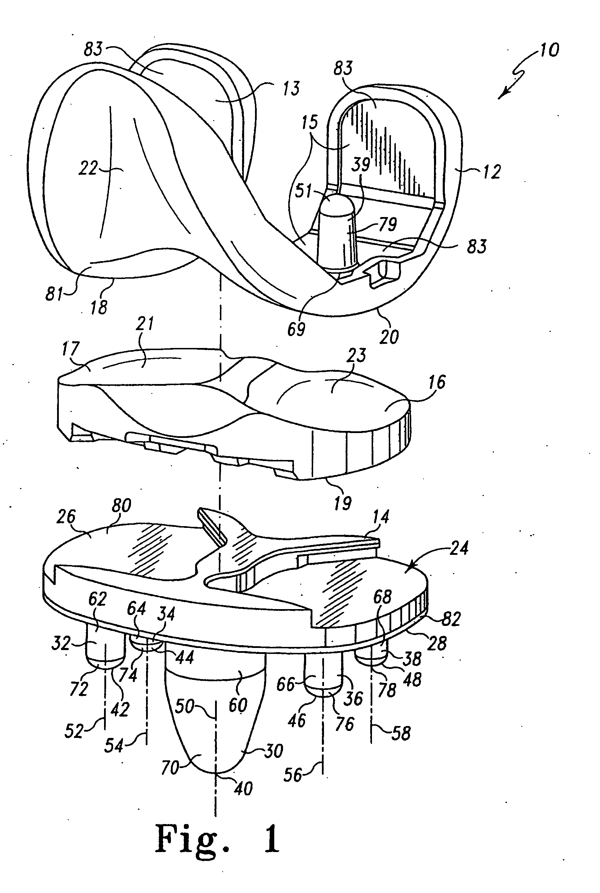

Referring to the drawings, FIG. 1 shows a knee prosthesis 10 which includes a femoral component 12, a tibial tray 14, and a bearing 16. The knee prosthesis 10 is a fixed bearing knee prosthesis, meaning that no movement is intended to occur between the tibial tray 14 and the bearing 16. It should be understood that the invention may also be applied to mobile bearing designs, such as rotating platform tibial trays, as well as to other joint prostheses.

The femoral component 12 includes two condylar articulation surfaces: a medial condyle articulation surface 18 and a lateral condyle articulation surface 20. These articulation surfaces 18, 20 are solid metal. The femoral component 12 is configured to be implanted into a surgically prepared end of the patient's femur (not shown), and is configured to emulate the configuration of the patient's natural femoral condyles. As such, the lateral condyle surface 20 and the medial condyle surface 18 are configured (e.g., curved) in a manner which mimics the condyles of the natural femur. The lateral condyle surface 20 and the medial condyle surface 18 are spaced apart from one another so as to define an intercondylar articulation surface 22 between them. The intercondylar articulation surface 22 defines a patella groove shaped to receive and bear against a patella implant component (not shown). The intercondylar articulation surface 22 may comprise solid metal.

The femoral component 12 also includes bone-engaging surfaces 13, 15 opposite the articulation surfaces 18, 20, 22. Some or all of the bone-engaging surfaces 13, 15 may comprise porous metal (as described below) conducive to bony ingrowth. Alternatively, the bone-engaging surfaces of the femoral component may include cement pockets to facilitate cementing the component to the bone.

The femoral component 12 of FIG. 1 is a cruciate retaining component, although it should be understood that the invention is applicable to cruciate substituting prosthetic knee systems as well.

The femoral component 12 may include features of standard, commercially available implants, such as those available from DePuy Orthopaedics, Inc., Warsaw, Indiana, as well as those available from other suppliers of prosthetic knee systems. The femoral component 12 may also include features disclosed in US-A-2010/036500, US-A-2009/032664, US-A-2009/0326667, US-A-2009/326666, and US-A-2009/326665.

The articulation surfaces of the femoral component 12 maybe constructed from a biocompatible metal, such as stainless steel, titanium, cobalt chromium alloy or titanium alloy, although other materials may also be used. Commonly used alloys include titanium alloy Ti-6A1-4. In one aspect of the present invention, the articulation surfaces 18, 20, 22 of the femoral component 12 comprise a titanium alloy (such as Ti-6A1-, for example) and the bone-engaging surfaces 13, 15 comprise titanium metal foam (such as a foam made of commercially pure titanium powder, 325 mesh (<45 µm), produced by a hydride-dehydride process and that meets the ASTM F-1580 standard, available from Phelly Materials, Inc., Bergenfield, New Jersey, Part No. THD325 for example) or a mix of such a powder with a compatible titanium alloy powder, such as Ti-6A1-4V. As discussed in more detail below, the titanium metal foam may comprise a titanium foam preform bonded to the solid titanium alloy through sintering.

As shown in FIG. 1, the bearing component 16 has a proximal articulation surface 17 and a distal mounting surface 19 opposite the proximal articulation surface 17. The proximal articulation surface 17 of the bearing 16 includes a medial bearing surface 21 configured to articulate with the medial condyle 18 of the femoral component 12 and a lateral bearing surface 23 configured to articulate with the lateral condyle 20 of the femoral component 12. The bearing component 16 is modular, and is assembled with the tibial tray 14 intraoperatively and secured thereto through a mechanical interlocking mechanism, as described in more detail below.

The bearing 16 may be made of a polymeric material. Suitable polymeric materials for the bearing 16 include ultrahigh molecular weight polyethylene (UHMWPE). The UHMWPE may comprise a cross-linked material, for example. Techniques for crosslinking, quenching, or otherwise preparing UHMWPE are disclosed in documents such as US-5728748, US-5879400, US-6017975, US-6242507, US-6316158, US-6228900, US-6245276 and US-6281264. The UHMWPE of the bearing material may be treated to stabilize any free radicals present therein, such as through the addition of an antioxidant such as vitamin E. Techniques for stabilizing UHMWPE with antioxidants are disclosed in, for example, US-A-2007/0293647 and US-A-2003/021216. It should be understood that the present invention is not limited to any particular UHMWPE material or to UHMWPE material for the bearing 16. It is expected that other materials for the bearing 16 are or will become available that will be useful in the invention.

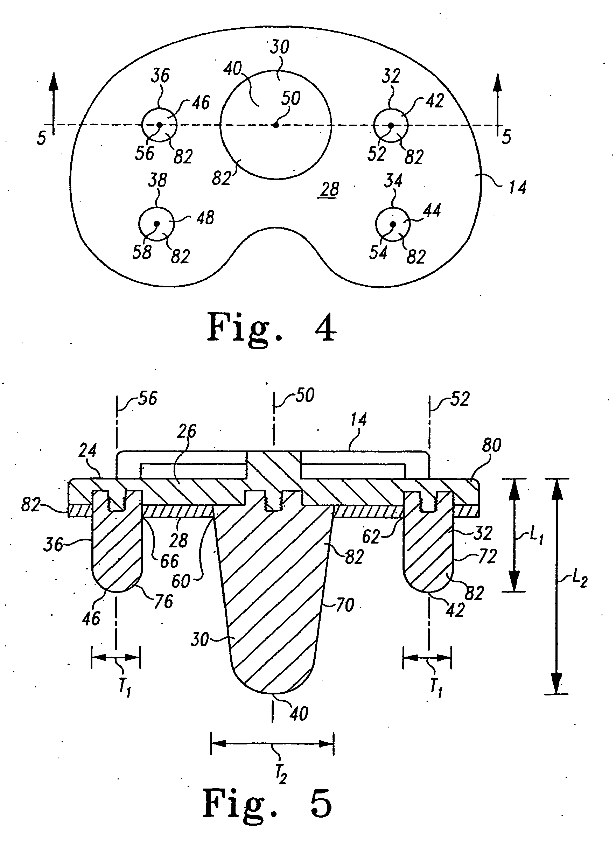

The tibial tray 14 includes a platform 24 having a solid metal proximal mounting surface 26 and an opposite distal bone-engaging surface 28. The tibial tray 14 also includes a plurality of extensions 30, 32, 34, 36, 38 extending distally from the distal bone-engaging surface 28 of the platform to distal ends 40, 42, 44, 46, 48 along longitudinal axes 50, 52, 54, 56, 58 intersecting the distal surface 28 of the platform 24. Each extension 30, 32, 34, 36, 38 has an axial length, shown, for example, as L₁ and L₂ in FIG. 5 and a thickness, shown, for example, as T₁ and T₂ in FIG. 5.

The femoral component 12 may also include extensions. For example, pegs may extend proximally from the bone-engaging surfaces 13, 15 of the femoral component 12. One such peg is shown in FIG. 1 at 39. This peg also has a thickness and a length.

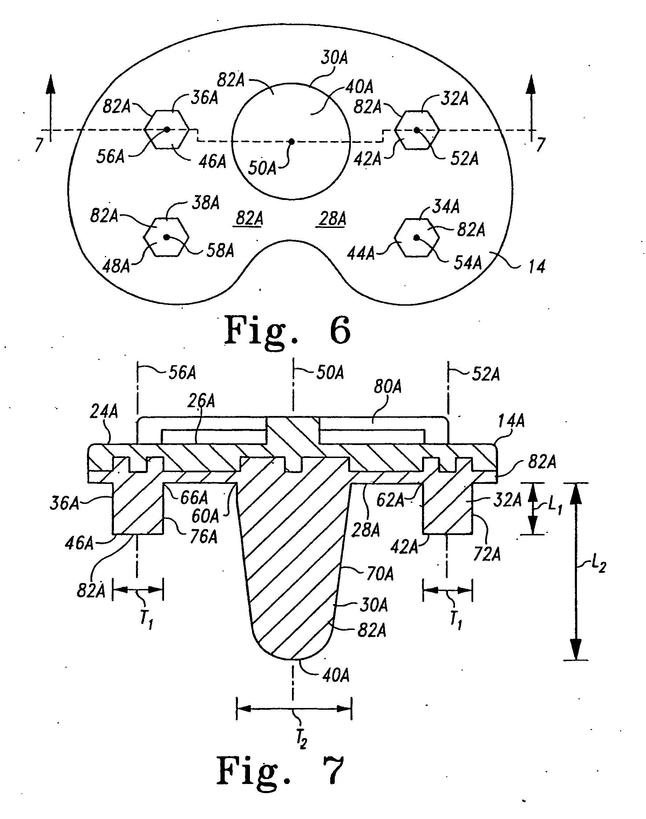

In the femoral component and tibial tray shown in the drawings, each extension 30, 32, 34, 36, 38, 39 extends outward from a junction with the bone-engaging surfaces 13, 15, 28 of their respective implant components 12, 14 to their opposite ends 40, 42, 44, 46, 48, 51. Examples of such junctions are shown in FIG. 1 at 69, in FIG. 5 at 60, 62 and 66 and in FIG. 7 at 60A, 62A, 66A. The extensions 30, 32, 34, 36, 38, 39 have exposed outer surfaces past the junctions; examples of such exposed outer surfaces are shown at 79 in FIG. 1, at 70, 72 and 76 in FIG. 5 and at 70A, 72A and 76A in FIG. 7.

The extensions 30, 32, 34, 36, 38 of the first and second tibial tray embodiments define a stem 30, 30A and four spaced pegs 32, 34, 36, 38, 32A, 34A, 36A, 38A. The stem 30, 30A and pegs 32, 34, 36, 38, 32A, 34A, 36A, 38A are configured to be implanted into a surgically prepared end of a patient's tibia (not shown) and are configured for stabilizing the tibial component 14, 14A when implanted in a bone of a patient. The stem 30, 30A is generally in the central sagittal plane of the tibial component, and the pegs 32, 34, 36, 38, 32A, 34A, 36A, 38A are spaced from the central sagittal plane of the tibial component.

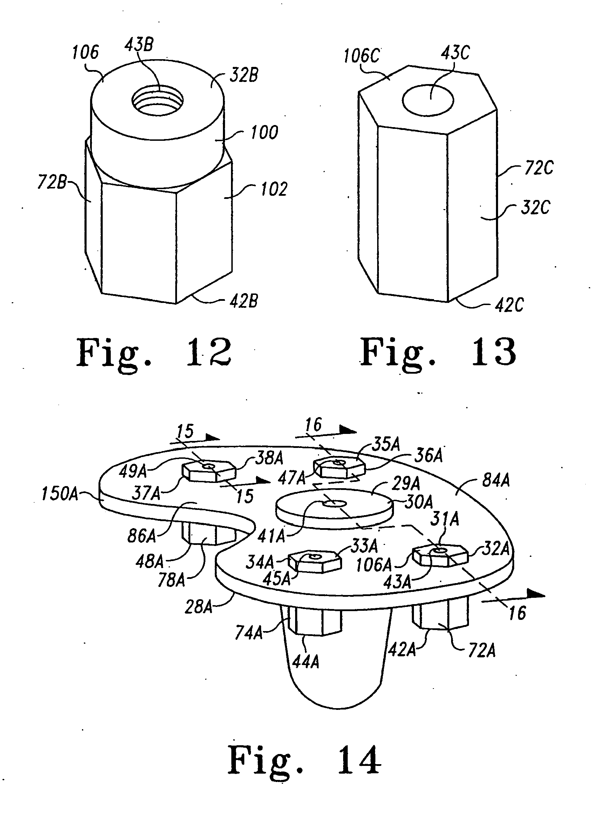

The stem 30, 30A may be shaped as a standard stem for tibial trays, tapering from the junction 60, 60A with the bone-engaging surface 28, 28A of the tibial tray 14, 14A to its distal end 40, 40A. Each of the tibial pegs 32, 34, 36, 38 in the embodiment of FIGS. 1, 4 and 5 is circular in transverse cross-section and end view. Other shapes may also be used for the pegs. The pegs may be tapered or cylindrical. The pegs may be a combination of shapes, such as a combination of cylindrical and hexagonal, as shown in FIG. 12 at 32B. Alternatively, the pegs may be hexagonal in cross-section and end view, as shown in FIG. 13 at 32 C. In FIGS. 12 and 13, the reference numbers are the same as those used in the description of the embodiment of FIGS. 1, 4 and 5 for similar parts, followed by the letters "B" and "C".

The distal end surfaces of the stem and pegs could be flat, spheroidal or some other shape. In the embodiment of FIGS. 1, 4 and 5, the free ends 40, 42, 44, 46, 48, 51 are generally spheroidal. In the embodiments of FIGS. 12 and 13, the distal ends 42B, 42C are flat. It should be understood that pegs and stems having other shapes might be used.

Another embodiment is shown in FIGS. 6 and 7, where the same reference numbers have been used as those used in describing corresponding or similar parts in the embodiment of FIGS. 1, 4 and 5, followed by the letter "A". As described in more detail below, in the embodiment of FIGS. 6 and 7, all of the extensions 30A, 32A, 34A, 36A, 38A are part of a single integral preform. The embodiments may share features as described above and below. Differences between the embodiments are described above and below.

The tibial trays 14, 14A illustrated in FIGS. 1 and 3 to 7 are composites of two materials; each tray 14, 14A includes solid metal portions 80, 80A and porous metal portions 82, 82A. The solid metal portions 80, 80A of the tibial trays 14, 14A define the proximal mounting surfaces 26, 26A of the platforms 24, 24A and bear against the distal mounting surface 19 of the bearing component 16 when assembled. The femoral component of FIG. 1 may also be a composite of a solid metal portion 81 and a porous metal portion 83, with the solid metal portion 81 defining the articulating surfaces 18, 20, 22.

The porous metal portions 82, 82A, 83 of the tibial tray 14, 14A and femoral component 12 define the distal bone-engaging surfaces 28, 28A of the tibial platform 24, 24A and the bone-engaging surfaces 13, 15 of the femoral component 12. These porous metal bone-engaging surfaces 13, 15, 28, 28A face the bone of the resected proximal surface of the tibial plateau and resected surfaces of the distal femur when implanted, and define a material that is conducive to bone ingrowth to allow for uncemented fixation of the tibial platform 24, 24A to the proximal tibia and the femoral component 12 to the distal femur. As described in more detail below, the porous metal portion 82, 82A of the tibial tray 14, 14A extends proximally from the distal bone-engaging surface 28, 28A and is sintered to the solid metal portion 80, 80A at a location between the distal bone-engaging surface 28, 28A and the proximal mounting surface 26, 26A of the platform 24, 24A. The femoral component 12 is similarly constructed, with the porous metal portion 83 sintered to the solid metal portion 81 at a location between the bone-engaging surfaces 13, 15 and the articulating surfaces 18, 20, 22.

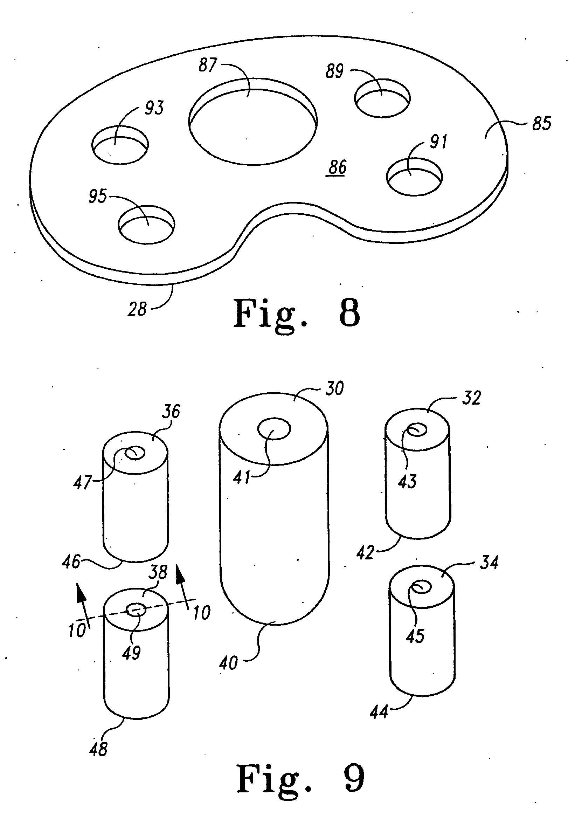

The porous metal portions 82, 82A, 83 of the tibial tray 14 and femoral component 12 may comprise preforms or a plurality of preforms. A first example of a set of porous metal preforms for a tibial tray 14 is shown in FIGS. 8 and 9. This set of porous metal preforms includes a base preform 85 with an upper surface 86 opposite from the distal bone-engaging surface 28. The upper surface 86 becomes the interface with the solid metal portion 80 of the tibial tray 14 when the porous metal base preform 85 is sintered to the solid metal portion 80 to make the tibial tray 14. As described in more detail below, the first illustrated base preform 85 includes a plurality of smooth cylindrical bores or openings 87, 89, 91, 93, 95 extending from the upper surface 86 to the distal bone-engaging surface 28.

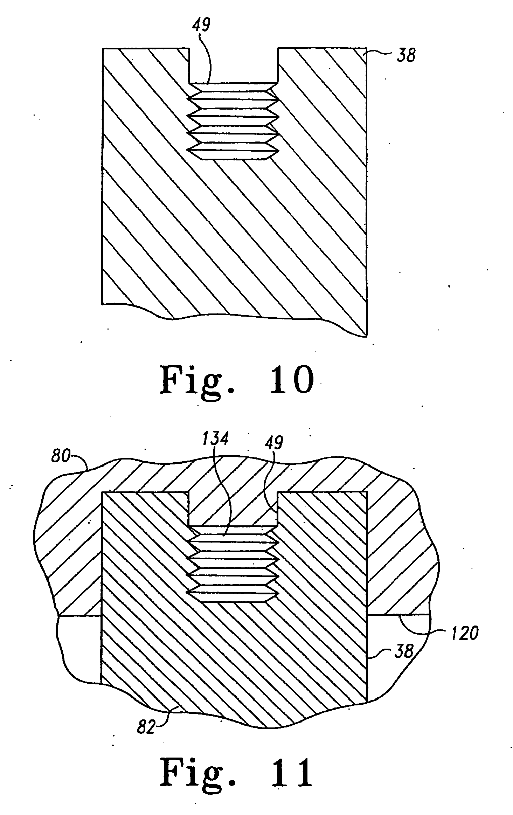

As shown in FIG. 9, the extensions 30, 32, 34, 36, 38 in the first set of porous metal preforms are discrete components, separate from the base preform 85 before being sintered together. The extension preforms are circular in transverse cross-section, with diameters substantially the same as the diameters of the bores 87, 89, 91, 93, 95 in the base preform 85. Portions of the extensions adjacent to the proximal ends of the extensions fit through the bores 87, 89, 91, 93, 95 and make contact with the walls of the base preform so that the preform 85 and extensions 87, 89, 91, 93, 95 may be sintered together. The proximal ends of the discrete extensions include blind bores 41, 43, 45, 47, 49 aligned along the longitudinal axes 50, 52, 54, 56, 58 of the extensions 30, 32, 34, 36, 38. The bores 41, 43, 45, 47, 49 are threaded in this embodiment. For clarity of illustration, FIG. 9 does not show the threads in these bores 41, 43, 45, 47, 49. An example of such a threaded bore 49 is shown in longitudinal cross-section in FIG. 10.

Other shapes of extensions may be used in combination with the base preform 85. For example, the extensions corresponding to the pegs may comprise a combination of a cylindrical portion and a portion that is hexagonal in transverse cross-section. Such a peg is shown in FIG. 12 at 32B; the cylindrical portion is shown at 100 and the hexagonal portion is shown at 102. This peg preform also has a flat end surface 42B opposite the end surface 106 that includes the threaded bore 43B.

Another example of an extension that may be used in the present invention is shown in FIG. 13 at 32C. In this example, the extension 32C is hexagonal in transverse cross-section and in end view. The extension includes two flat ends 42C, 106C with a blind bore 43C in one end 106C. In this example, the blind bore 43C is not threaded. Instead, the walls of the bore 43C define a Morse taper bore for receipt of a Morse taper post as described in more detail below. The walls defining the bore 43C may be tapered at an angle of, for example 3 to 5°. The bore is widest at the end 106C and most narrow between the end 106C and the end 42C. Peg preforms such as those illustrated in FIG. 13 could be used with a tibial platform preform similar to that illustrated in FIG. 8, except the bores or holes 89, 91, 93, 95 would have hexagonal shapes to receive and hold the extension 32C.

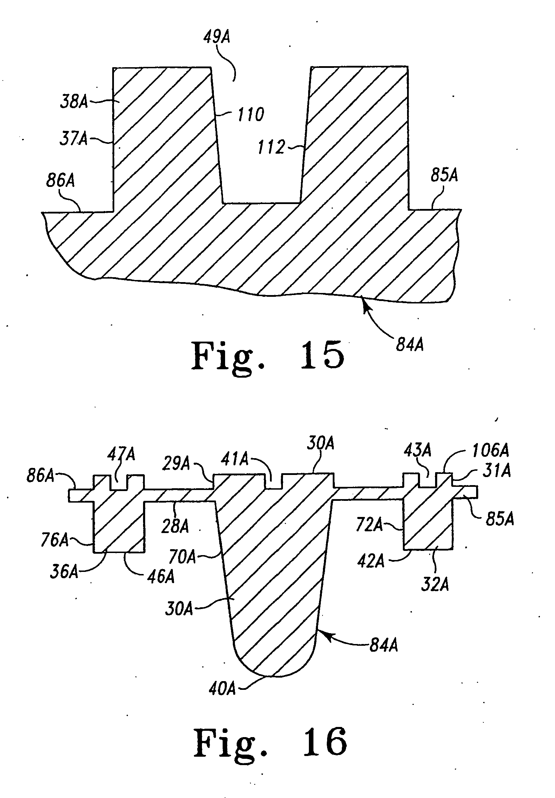

An example of a porous metal preform utilizing extensions shaped like those of FIG. 13 is shown in FIG. 14. In this example, the porous metal preform 84A includes a base portion 85A and integral extensions 30A, 32A, 34A, 36A, 38A. The extensions 32A, 34A, 36A, 38A correspond with pegs and the extension 30A corresponds with the stem of the tibial tray. In this embodiment, the extension 30A corresponding with the stem is circular in transverse cross-section, although it should be understood that other shapes may be used. On the proximal side of the base 85A, an annular raised portion 29A, 31A, 33A, 35A, 37A of each extension extends above the planar proximal surface 86A of the base 85A. Each extension includes a longitudinal bore or opening 41A, 43A, 45A, 47A, 49A. As discussed above with respect to FIG. 13, in this embodiment, the longitudinal bores or openings 41A, 43A, 45A, 47A, 49A are Morse taper bores tapering in a distal direction. An enlarged cross-sectional view of one of the annular raised portions 37A and its associated bore 49A is shown in FIG. 15 as an illustrative example; the walls 110, 112 defining the tapered bore 49A may be angled at any suitable angle for a Morse taper bore, such as, for example, 3 to 5°. The annular projections 29A, 31A, 33A, 35A, 37A may be cylindrical in shape, like that shown at 29A, or may have some other shape, such as the hexagonal shape (in transverse cross-section and plan view) like those shown at 31A, 33A, 35A and 37A.

A cross-section of the porous metal preform 84A is shown in FIG. 16 as an example. The porous metal preform 84A can be made as a single, integral piece in the moulding process and can be otherwise processed in standard ways, such as by machining to create particular features. FIG. 7 illustrates the preform 84A of FIGS. 14 to 16 in combination with a solid metal portion 80A to form the tibial tray 14A.

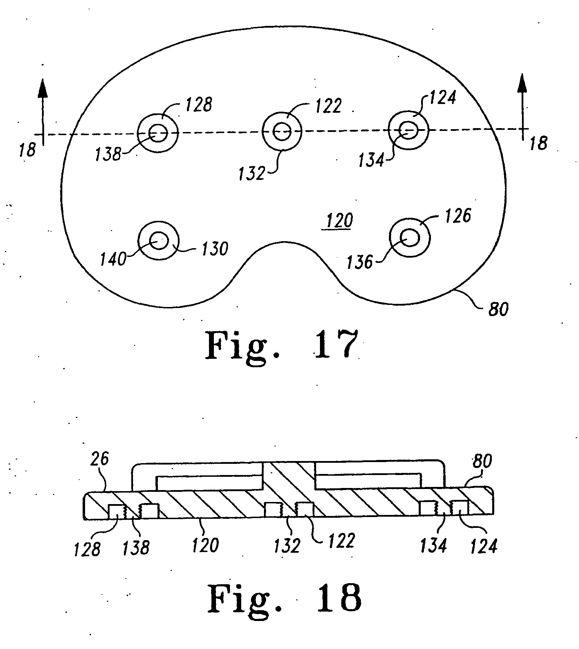

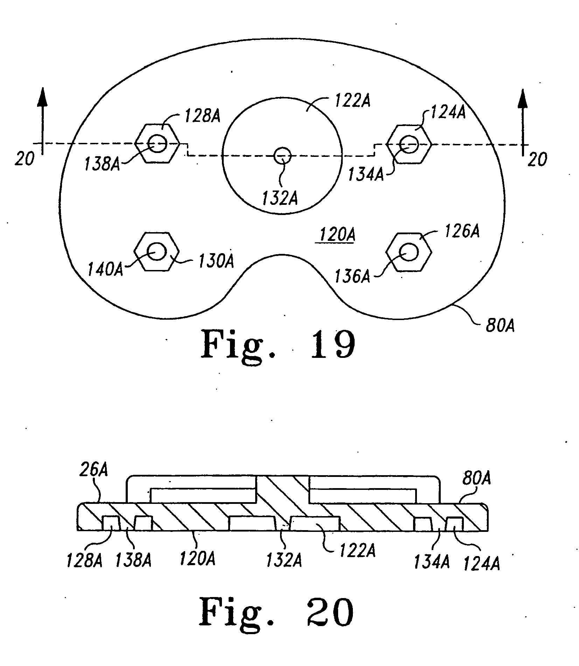

Referring back to the solid metal portion 80 of the tibial tray 14, a first example of a distal surface 120 of the solid metal portion is shown in FIG. 17. The distal surface 120 is opposite the proximal mounting surface 26 of the platform 24 of the tibial tray 14 of FIG. 1. As there shown, the distal surface 120 includes a plurality of recesses 122, 124, 126, 128, 130. A stud 132, 134, 136, 138, 140 is present within each recess 122, 124, 126, 128, 130. The distal surface of a second example of the solid metal portion 80A of a tibial tray is shown in FIG. 19. As there shown, the distal surface 120A also includes a plurality of recesses 122A, 124A, 126A, 128A, 130A. A stud 132A, 134A, 136A, 138A, 140A is present within each recess 122A, 124A, 126A, 128A, 130A.

The recesses 122, 124, 126, 128, 130 in the embodiment of FIGS. 17 and 18 are configured to receive the cylindrical ends of the extensions 30, 32, 34, 36, 38 and the studs 132, 134, 136, 138, 140 are threaded and complementary to the threaded bores 41, 43, 45, 47, 49 so that the extensions 30, 32, 34, 36, 38 may be threaded onto the studs 132, 134, 136, 138, 140 to mount the extensions to the studs 132, 134, 136, 138, 140. Preferably, the recesses 122, 124, 126, 128, 130 and extensions 30, 32, 34, 36, 38 are shaped so that there is metal-to-metal contact between the outer surfaces of the extensions 30, 32, 34, 36, 38 and the walls defining the recesses 122, 124, 126, 128, 130 so that the extensions 30, 32, 34, 36, 38 may be sintered to the solid metal portion 80.

The recesses 122A, 124A, 126A, 128A, 130A in the embodiment of FIGS. 19-20 are configured to receive the annular raised portions 29A, 31A, 33A, 35A, 37A of the preform 84A (or ends of the extensions 30A, 32A, 34A, 36A, 38A) and the studs 132A, 134A, 136A, 138A, 140A are tapered and complementary to tapered bores 41A, 43A, 45A, 47A, 49A so that the preform 84A may be frictionally mounted onto the studs 132A, 134A, 136A, 138A, 140A. The recesses 122A, 124A, 126A, 128A, 130A and annular raised portions 29A, 31A, 33A, 35A, 37A have complementary shapes (hexagonal in transverse cross-sections) so that there is metal-to-metal contact between the annular raised portions 29A, 31A, 33A, 35A, 37A and the walls defining the recesses 122A, 124A, 126A, 128A, 130A so that the preform 84A may be sintered to the solid metal portion 80A.

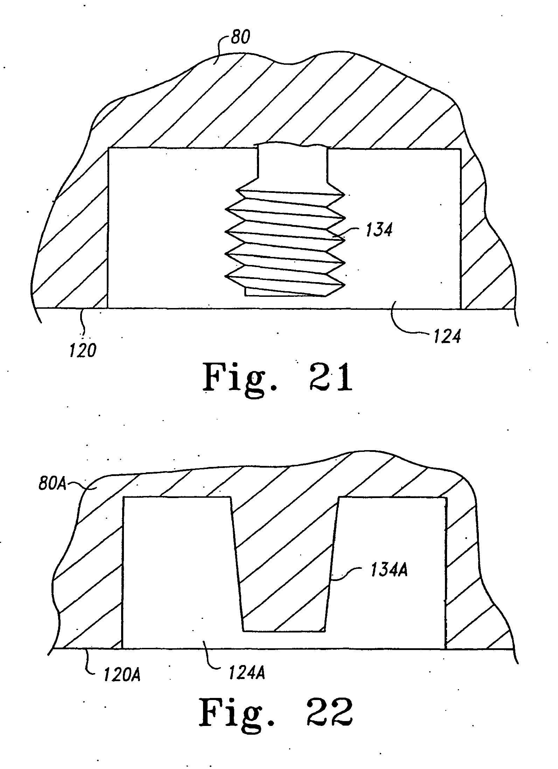

Examples of configurations for studs are shown in FIGS. 21 and 22. The studs may be threaded, such as stud 134 shown in FIG. 21 to allow for a threaded connection between the studs and the corresponding threaded bores of the extensions; such a connection is shown in FIG. 11, where threaded stud 134 is shown connected with extension 38 through such a threaded connection.

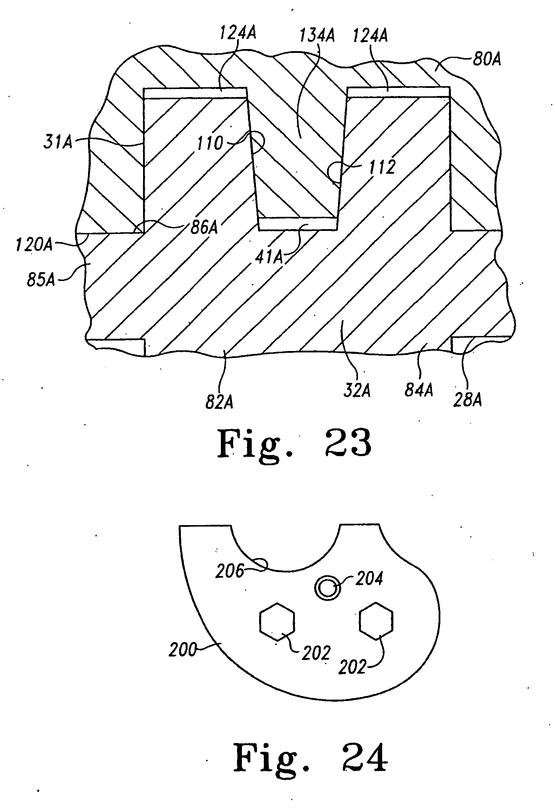

The studs may alternatively comprise Morse taper posts having a Morse taper (generally about 3 to 5°); such a stud is shown in FIG. 22 at 134A. Generally, the studs are sized, shaped and positioned to be received within the Morse taper bore (generally about 3 to 5 °) of a corresponding extension so that the extensions may be mounted on the studs. Such a connection is shown in FIG. 23, where Morse taper stud 134A is shown engaged with Morse taper bore 41A in preform 84A. It should be understood that the mounting mechanisms shown in FIGS. 21 and 22 are provided as examples only; other suitable structures may be used for mounting the extensions 30, 32, 34, 36, 38 and preform 84A to the corresponding solid metal portion 80, 80A.

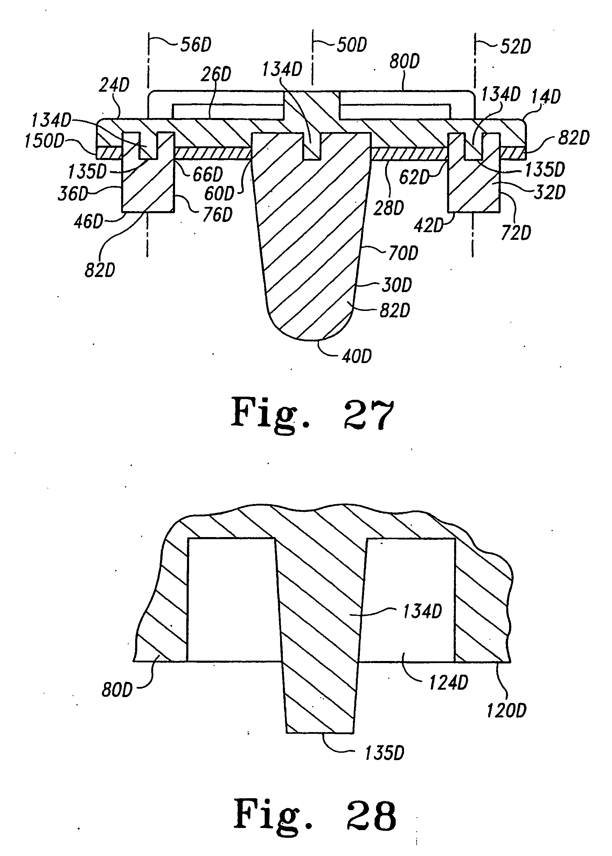

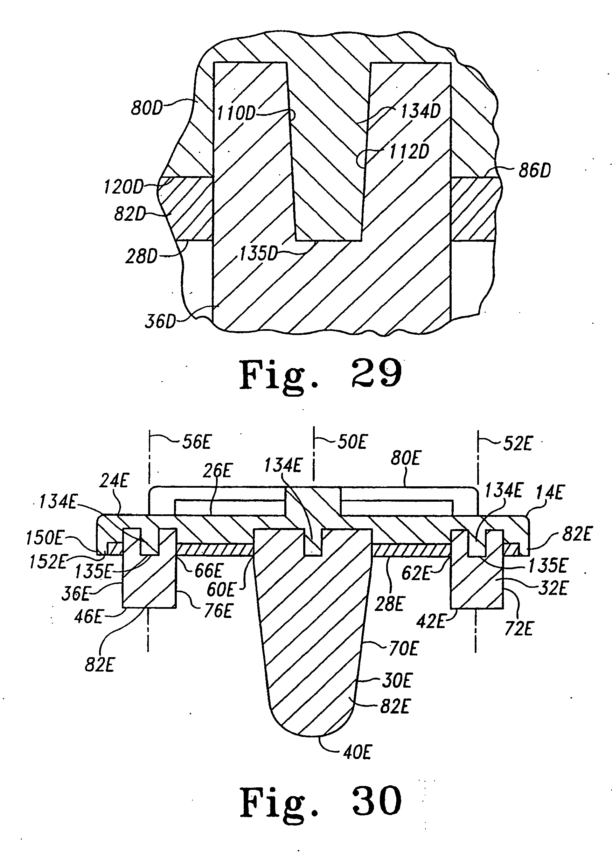

In the embodiments of FIGS. 5, 7, 11, 18 and 20 to 23 the studs 134, 134 have free ends 135, 135A that do not extend beyond the plane of the distal surface 120, 120A of the solid metal portion 80, 80A of the tibial tray 14, 14A. An alternative embodiment of a tibial tray with longer studs is shown in FIGS. 27 to 29, where the same reference numbers have been used as those used in describing corresponding or similar parts in the embodiments of FIGS. 1, 4 to 7, 11, 18 and 20 to 23 followed by the letter "D". In the embodiment of FIGS. 27 to 29, the free ends 135D of the studs extend beyond the plane of the distal surface 120D of the solid metal portion 80D of the tibial tray 14D. When assembled with the porous metal portion 82D as shown in FIGS. 27 and 29, the free ends 135D of the studs extend to the plane of the bone-engaging surface 28D of the porous metal portion of the tibial tray 14D.

The configuration of the proximal mounting surface 26, 26A of the solid metal portion 80, 80A of the tibial tray 14, 14A may vary depending on the type of implant. For example, if the prosthesis is a rotating platform type of mobile bearing knee prosthesis, the proximal mounting surface 26, 26A of the tibial tray 14, 14A and the distal mounting surface 19 of the bearing 16 will be smooth to allow for rotation of the bearing on the proximal mounting surface 26, 26A of the tibial tray 14, 14A. The embodiment shown in FIG. 1 is a fixed bearing design; the proximal mounting surface 26 of the tibial tray 14 and the distal mounting surface 19 of the bearing 16 in this embodiment include complementary locking features that eliminate or at least minimize any relative movement between the bearing 16 and the tibial tray 14 when these components are assembled. These complementary locking features in this embodiment include pedestals 154, 158, tabs 160, 162 and recesses 178, 180 on the distal mounting surface 19 of the bearing 16 and buttresses 184, 186 and undercuts 194, 196, 198 on the proximal mounting surface 26 of the solid metal portion 80 of the tibial tray 14. Detailed descriptions of this and other designs for fixed bearing tibial trays may be found in, for example, US-7628818 and US-A-2009/0082873.

Preferably, the solid metal portion 80, 80A of the tibial tray 14, 14A is a solid metal preform, made from a standard titanium metal alloy. A suitable alloy for this purpose is Ti-6A1-4V. This alloy is advantageous in that it may be sintered to a porous metal portion made from commercially pure titanium powder. This same material may be used for the solid metal portion of the femoral component 12 as well. It should be understood that some of the advantages of the present invention may be achieved with other materials, including for example a standard cobalt chromium molybdenum alloy.

Preferably, the porous metal portion 82, 82A of the tibial tray 14, 14A is a titanium metal foam. Such a foam may be made as disclosed in US-A-2008/0199720, US-A2010/0098574, US-A-2009/0326674 and US-A-2009/0292365. The titanium metal powder used to make the porous metal portion 82, 82A may comprise commercially pure titanium powder ( such as a titanium powder, 325 mesh (<45 µm), produced by a hydride-dehydride process and that meets the ASTM F-1580 standard, available from Phelly Materials, Inc., Bergenfield, New Jersey, Part No. THD325 for example) or a mix of such a powder with a compatible titanium alloy powder, such as alloy Ti-6A1-4V. This material is advantageous in that it can be sintered to a titanium alloy such as Ti-6A1-4V. It is expected that other grades of commercially pure titanium may be used as well and that other powder metal materials may be available or developed in the future that can provide at least some of the advantages of the present invention.

Although titanium foam is preferred, some of the advantages of the present invention may be achieved with alternative materials as well. One example of a suitable alternative material is tantalum porous metal, for example as disclosed in US-5282861. Another example of an alternative is a solid metal body made from an implantable metal such as stainless steel, cobalt chromium alloy, titanium, titanium alloy or the like and with a porous coating disposed on both the bone-engaging surface and the surface engaging the polymer portion of the tibial tray. One type of porous coating which may be used as the porous portion 82, 82A of the tibial tray 14, 14A is that used on products sold by DePuy Orthopaedics Inc of Warsaw Indiana under the trade mark Porocoat. The porous metal preform 84A may be made using any of the process described above or using other processes.

To make the tibial tray 14, 14A of the invention, the solid metal portion 80, 80A may be made as a solid metal preform by conventional methods, such as by casting, machining or some combination of casting and machining. Such processes may also be used to make a solid metal preform for the femoral component 12. For either the tibial tray 14, 14A or the femoral component 12, the recesses 122, 124, 126, 128, 130, 122A, 124A, 126A, 128A, 130A, and posts or studs 132, 134, 136, 138, 140, 132A, 134A, 136A, 138A, 140A may be machined into the solid metal preforms. For studs of the type shown in FIG. 21, threads may be formed in the studs 132, 134, 136, 138, 140 as well. For studs of the type shown in FIG. 22, the outer surface of the studs 132A, 134A, 136A, 138A, 140A may be shaped to define a Morse taper post.

It is expected that the articulation and mounting surfaces 18, 20, 26 of the solid metal portions of the femoral and tibial components 12, 14 may be treated to increase the lubricity, such as through Type II hard anodization.

The porous metal portion 82, 82A of the tibial tray 14, 14A and femoral component 12 may be made by moulding the desired shape, for example using processes as disclosed in US-A-2008/0199720 and US-A-2010/098574. Preforms so made can have, for example, a bulk porosity (or percent open area or void space) of from about 60% to about 85% (preferably about 65% to about 75%) as measured by volume, the forced intrusion of liquid mercury, and cross-section image analysis. This porosity/void space corresponds with a preform having a density of 15 to 35% (preferably 25 to 35%) of theoretical density for a similarly sized and shaped solid metal component. It should be understood that the porosity can be a product of various factors in the manufacturing process, such as the size of pore forming agent used. The resultant titanium metal foam may be treated to increase its roughness, such as by etching or blasting, as discussed in more detail below.

The moulds used for preparing the porous metal portion 82A may be shaped so that the resultant product defines a single, integral porous metal preform 84A such as that shown in FIG. 16. Such a preform can be used to make a tibial tray 14A such as that shown in FIGS. 6 and 7. Alternatively, a plurality of moulds may be provided to make individual and discrete extensions 30, 32, 34, 36, 38 and an individual and discrete base 85 for the embodiment of FIGS. 4, 5, 9 and 9. The bores 41, 43, 45, 47, 49, 41A, 43A, 45A, 47A, 49A in these components may be formed as part of the moulding process or machined into the finished metal foam construct. For extensions of the type shown in FIGS. 5 and 9 to 12, threads may be formed in the walls defining the bores 41, 43, 45, 47, 49. For extensions of the type shown in FIGS. 7, 13 to 16 and 23, the walls defining the bores 41A, 43A, 45A, 47A, 49A may be tapered to define Morse taper bores.

The porous metal portion 82, 82A of the implant component and the solid metal portion 80, 80A of the implant component may then be assembled. For example, for an implant component of the type shown in FIGS. 6 and 7, the integral preform 84A may be pressed onto the distal surface 120A of the solid metal portion 80A, with the Morse taper studs 132A, 134A, 136A, 138A, 140A of the solid metal portion 80A pushed into the Morse taper bores 41A, 43A, 45A, 47A, 49A of the preform 84A, and with the annular raised portions 29A, 31A, 33A, 35A, 37A of the porous metal preform 84A received in the recesses 122A, 124A, 126A, 128A, 130A surrounding the studs 132A, 134A, 136A, 138A, 140A of the solid metal portion or preform 80A, as shown in FIGS. 7 and 22. The Morse taper frictional connection between the studs and the bores should hold the assembly together until sintering is complete. For an implant component of the type shown in FIGS. 4 and 5, each porous metal extension 30, 32, 34, 36, 38 may be individually assembled with the solid metal base 80 by threading the threaded bore 41, 43, 45, 47, 49 of each porous metal extension 30, 32, 34, 36, 38 onto the threaded stud 132, 134, 136, 138, 140 of the solid metal portion or preform 80 until the annular end of the extension is received in the recess 122, 124, 126, 128 130 surrounding the stud 132, 134, 136, 138 as shown in FIG. 11. This threaded connection between the studs 132, 134, 136, 138 and the bores 41, 43, 45, 47, 49 should hold the assembly together until sintering is complete. It should be understood that the Morse taper connection and threaded connection described above are two examples of complementary structures for connecting the porous metal extensions to the solid metal portion of the tray; other types of connections may be used.

The assembly of the solid metal portion 80, 80A, 81 and the porous metal portions 82, 82A, 83 may then be sintered together to form the final tibial tray 14, 14A or femoral component 12. Sintering may be accomplished utilizing the same temperatures and times used to form the porous metal portion. For example, as disclosed in US-A-2008/199720, the assembly may be sintered under the following conditions to form the final implant component: heating at temperatures of from about 1149°C to about 1482°C (2100°F to 2700°F), preferably about 1371 °C (2500°F) for about 2 h to about 10 h (preferably about 3 h to about 6 h). The sintered part may then be cooled following an appropriate cooling cycle.

For both the femoral and tibial components, once assembled, the porous metal portion 82, 82A, 83 defines the bone-engaging surfaces 13, 15, 28, 28A of the implant component 12, 14, 14A. In addition, for both the femoral and tibial components, the solid metal portions 80, 80A, 81 contact the bearing 16, both on the mounting side 19 and the articulation side 17.

As mentioned above, in some situations, it may be desirable to treat the porous metal portion 82, 82A, 83 to selectively increase the roughness of some or all of the bone-engaging surfaces. The porous metal portion 82, 82A, 83 may be treated through etching or blasting, for example, to increase the roughness of the outer surface, for example using a technique such as those as disclosed in US-A-2009/0326674 and US-A-2009/292365. Although the etching and blasting techniques disclosed in those documents are advantageous for use with titanium metal foams, it should be understood that the techniques disclosed in these documents are provided as examples only. Such roughening is expected to make the treated surfaces more conducive to bone ingrowth to improve ultimate fixation of the components.

A variety of other techniques are known for treating porous metal implants and may be applied to the present invention. For example, calcium phosphate coatings (such as hydroxyapatite) may be applied to some or all of the porous portions of the embodiments of the present invention, with or without additional therapeutic agents, as disclosed in US-A-2006/0257358 . Alternatively, electrophoretic deposition of a material such as calcium phosphate may be used.

As disclosed in US-A-2009/292365, porous metal samples (both commercially pure titanium and Ti-6A1-4V) were machined in the green state and the static coefficients of friction with polymer bone analogues for the surfaces were found to be 0.52 for commercially pure titanium and 0.65 for Ti-6A1-4V, with standard deviations of 0.1. In contrast, porous metal components of the same materials that were blasted as taught in that document had average static coefficients of friction with polymer bone analogues of 0.72-0.89 for commercially pure titanium and 1.09 to 1.35 for Ti-6A1-4V. As described in that document, these tests were performed using a polymer bone analogue having a density of about 320 kg.m⁻³ (20 lb.ft⁻³). One example of a bone analogue material is available from General Plastics Manufacturing Co. (Tacoma, WA), identified using the catalogue number FR-4520. It is said to be a "rigid, closed-cell polyurethane foam" with a density of 320 kg.m⁻³ (20 lb.ft⁻³). The friction test was performed using a "sled on a plane" method. The "sled" consisted of 19 mm × 19 mm (0.75 in × 0.75 in) square metallic matrix samples. Each "plane" was a milled sample of a rigid, closed-cell polyurethane foam with a density of 320 kg.m⁻³ (20 lb.ft⁻³) (as sold under the trade mark Last-A-Foam 6720 by General Plastics Manufacturing Company, Tacoma, WA). Each sled was connected to a 250 N load cell by 44.5 N (10 lb) monofilament line and pulled at 10 mm.min⁻¹ for 20 mm (0.8 in). A weight was placed on the sled to create a normal force of 30 N. The static friction coefficient was calculated from the maximum force recorded before the first 0.5 N drop in force.

Profile parameters of the test samples are also provided in US-A-2009/292365 pursuant to ISO 4287 (1997). As there shown the Pa, Pp, Pt and Pq values (as defined in the patent document) for the samples all at least doubled for the blasted samples as compared to the machined samples with no blasting.

One application of the etching and blasting roughening techniques of the above-identified patent documents is to roughen the porous metal portions 82, 82A, 83 of the tibial tray 14, 14A and femoral component 12. In addition, it may be advantageous to selectively roughen certain surfaces of the porous metal portion 82, 82A, 83 while leaving other surfaces in their as-machined state, with lower roughnesses. Specifically, to facilitate removal of either the tibial tray 14, 14A or the femoral component 12 from the bone in revision surgery, it may be desirable to discourage bone ingrowth at the distal ends 40, 42, 44, 46, 48, 40A, 42A, 44A, 46A, 48A of the tibial extensions and proximal ends 51 of the femoral extensions 39. This may be accomplished by selectively roughening the distal bone-engaging surface 24, 24A of the platform and the outer surfaces of the extensions 30, 32, 34, 36, 38, 30A, 32A, 34A, 36A, 38A at the junctions 60, 62, 66, 69, 60A, 62A, 66A and adjacent surfaces while leaving the ends 40, 42, 44, 46, 48, 40A, 42A, 44A, 46A, 48A opposite the junctions 60, 62, 66, 69, 60A, 62A, 66A (and some adjacent surfaces if desired) in the as-machined state.

For example, a tibial tray made according to this aspect of the invention may have a stem 30, 30A and pegs 32, 34, 36, 38, 32A, 34A, 36A, 38A with distal surfaces 40, 42, 44, 46, 48, 40A, 42A, 44A, 46A, 48A having a coefficient of static friction (with a polymer bone analogue comprising rigid closed-cell polyurethane foam with a density of about 320 kg.m⁻³ (20 lb.ft⁻³)) less than 0.7; the outer surfaces of these pegs 32, 34, 36, 38, 32A, 34A, 36A, 38A and stem 30, 30A near the junctions 60, 62, 66, 60A, 62A, 66A may have coefficients of static friction (with a polymer bone analogue comprising rigid closed-cell polyurethane foam with a density of about 320 kg.m⁻³ (20 lb.ft⁻³)) of more than 0.7. For pegs 32A, 34A, 36A, 38A of the type shown in FIGS. 7, 12 to 14 and 16, the flat distal surface 42A, 44A, 46A, 48A may have a lower coefficient of friction; for an extension of the type illustrated in FIGS. 1, 3 to 5 and 9, all or part of the spheroidal distal end may have a lower coefficient of friction. Similar results are expected to be obtained with selective etching of the extensions. Alternatively, the surfaces of the porous metal portion 82, 82A where bone ingrowth is undesirable may be machined, milled, polished or otherwise smoothed to reduce the roughness and/or porosity of the surface. Machining, milling, polishing or smoothing can be expected to close some or all of the pores and lower the coefficient of friction along the surface. For example, the surfaces where bone ingrowth is undersirable may be machined with a standard carbide tip rotating at a standard speed, such as 600 rpm. Machining may be carried on until the surface is smeared and has a solid rather than porous appearance; about 0.38 mm (0.015 inch) of material may be removed in this process. It should be understood that a commercial manufacturing process may be run under different parameters. Machining, milling, polishing or smoothing can be accomplished when the component is in the green state, before sintering, after sintering, or both before and after sintering.

Alternatively, pores may be selectively filled with metal. As another alternative, when moulding the porous metal portion of the implant or the pegs and stem, or when sintering the solid metal and porous metal portions together, solid metal pieces may be sintered to the free ends of the pegs and stems. Another alternative would include moulding a non-porous biocompatible polymer cap to the ends of the extensions; an example of such a polymer is polyetheretherketone (PEEK).

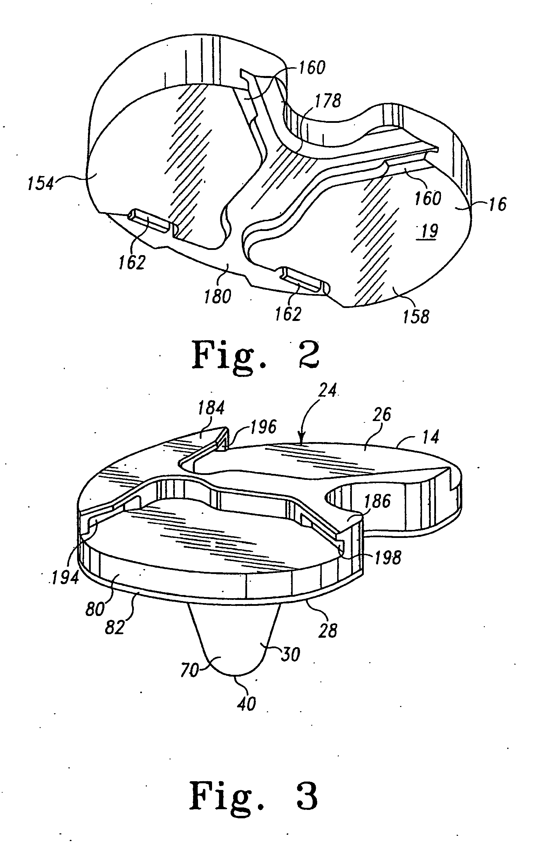

The porosity and roughness of other surfaces may also be modified. Considering the embodiment of FIGS. 1 and 3, for example, there are surfaces of the porous portion 82 that are not intended to engage bone or another part of the implant component. An example of such a surface is exposed peripheral surface 150 of the porous portion 82 of the tibial tray 14. This exposed peripheral surface 150 extends generally perpendicularly from the distal bone-engaging surface 28 to the upper surface 86 of the porous base 85 in the embodiment of FIGS. 1, 3 and 5. At least some of this exposed peripheral surface can be expected to be engaged by soft tissue when implanted. If this exposed peripheral surface is rough, adjacent soft tissue could be irritated when the tray is implanted. Accordingly, it may be preferable to smooth these exposed peripheral surfaces, or any surface that may engage soft tissue instead of bone or another portion of the implant. Any of the methods described above could be used. For example, the exposed peripheral surfaces could be machined with a carbide bit as described above. The coefficient of static friction of such a surface is expected to be no greater than those reported in US-A-2009/292365 for metal foam samples machined in the green state and not subjected to any roughening treatment (0.52 for commercially pure titanium and 0.65 for Ti-6A1-4V, with standard deviations of 0.1). Profile parameters of the peripheral exposed surfaces are also expected to be no rougher than the Pa, Pp, Pt and Pq values (as defined in US-A-2009/292365) for the metal foam samples machined in the green state. It is anticipated that the machining parameters could be adjusted to optimize the surface finishes of the peripheral exposed surfaces and distal surfaces 40. The exposed porous metal surfaces perpendicular to the bone-engaging surfaces of the femoral component 12 may be similarly treated.

An alternative embodiment of a tibial tray is shown in FIG. 30, where the same reference numbers have been used as those used in describing corresponding or similar parts in the embodiments of FIGS. 1, 4 to 7, 11, 18 and 20 to 23 followed by the letter "E". In this embodiment, the periphery of the solid metal portion 80E includes a rim 152 that extends to the plane of the bone-engaging surface 28E. In this embodiment, the rim 152 defines a pocket in which the porous metal base 85E is received so that the exposed peripheral surface 150E comprises solid metal. In this embodiment, the tibial tray may be made from a base component, such as a cast component, with pockets configured for cemented fixation, and the pockets could be filled with porous metal, such as a titanium foam, and then sintered.

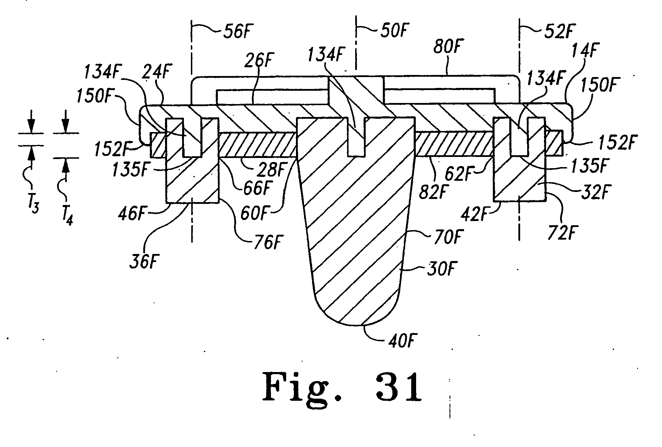

Another alternative embodiment of a tibial tray is illustrated in FIG. 32, where the same reference numbers have been used as those used in describing corresponding or similar parts in the embodiments of FIGS. 1, 4 to 7, 11, 18, 20 to 23 and 30 followed by the letter "F". In this embodiment, the periphery of the solid metal portion 80E includes a rim 152F that extends to a plane above the plane of the bone-engaging surface 28F. In this embodiment, the rim 152F defines a pocket in which a portion of the porous metal base 85F is received. In this embodiment, the porous metal base 85F is recessed from the periphery of the tibial tray to eliminate contact between the porous metal and soft tissue. Thus, the exposed peripheral surface 150F comprises solid metal. In this embodiment, the tibial tray may be made from a base component, such as a cast component, with pockets configured for cemented fixation, and the pockets could be filled with porous metal, such as a titanium foam, and then sintered. The pockets defined by the rim 152F have a depth shown at T₃ in FIG. 31, and the porous metal base 85F has a thickness shown as T₄ in FIG. 31. T₄ is greater than T₃ to ensure that the bone-engaging surface 28F stands proud so as to ensure that the surface 28F fully engages and transfers load to the underlying bone.

Bone loss on the proximal tibia or distal femur can make it difficult to properly position and support the tibial component 14, 14A or femoral component 12 of the implant system 10 on the bone surface. The prior art has addressed this problem through the use of wedges or augments. Generally, the wedge or augment is placed between part of the bone-engaging surface of the implant component and part of the bone to support part of the implant component on the bone by augmenting part of the bone.

Due in part to the fact that the size, shape and anatomy of virtually every patient is different, and the variability in the location and amount of bone loss on the proximal tibia, an extensive number of a variety of wedges and augments have been made available to the orthopaedic surgeon. For example, a typical surgical kit will include tibial wedges of different thicknesses and different configurations for use on either the medial or the lateral sides of the tibia.

In the present invention, the prosthetic knee system or kit 10 may include wedges or augments for both the femoral and tibial sides of the system. These augments may comprise porous metal, and more particularly, a porous metal foam of the same material and made under the same conditions as those discussed above for the porous metal portions 82, 82A, 83 of the tibial trays 14, 14A and femoral components 12.

For the femoral side, augments may have features such as those disclosed in US-5984969 and US-6005018. For the tibial side, augments may have features such as those disclosed in US-5019103 and US-7175665.

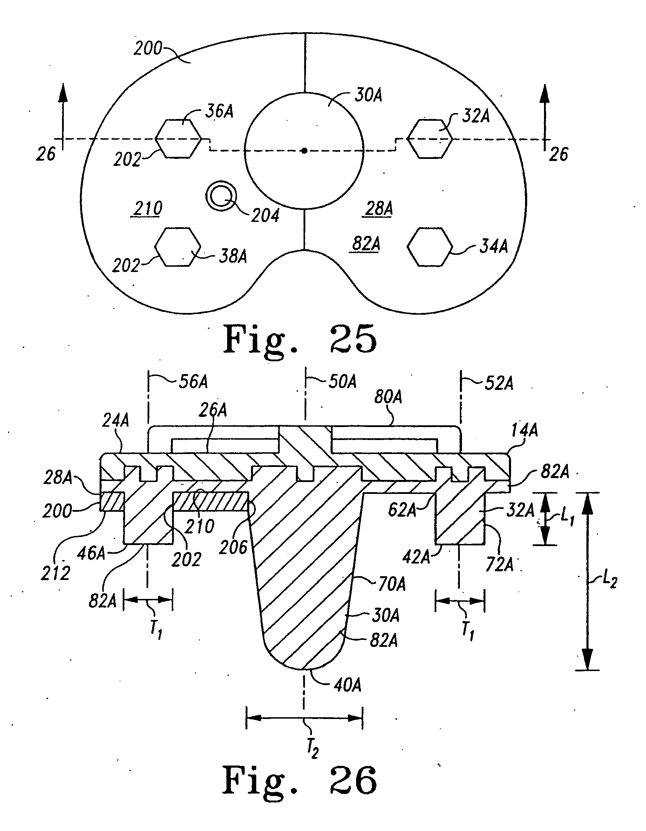

FIG. 24 shows a tibial augment 200 which is made of porous metal across its entire length, width and thickness. The augment 200 includes through-bores 202 sized and shaped to receive portions of pegs or extensions (such as pegs 32, 34, 36, 38, 32A, 34A, 36A, 38A) that may be present, and may be mounted to the porous metal portion 82, 82A of the tibial tray as shown in FIGS. 25 and 26. Frictional engagement of the augment and the pegs or extensions and the porous metal portion of the tray may be sufficient to fix the augment to the tray; otherwise, the augment 200 may include additional through-bores sized and shaped to receive screws (not shown) for fixing the augment 200 to the tibial tray 14, 14A; an illustrative through bore is shown at 204 in FIGS. 24 and 25. The augment 200 may also include a recess such as recess 206 to accommodate any stem (such as stem 30, 30A) on the tibial tray 14. Complementary blind bores may be provided in the tibial tray to receive parts of the screws. The bores in the tibial tray may be threaded, and may be provided in the porous metal portion 82, 82A or may extend through the porous metal portion 82, 82A and into the solid metal portion 80, 80A. The surfaces defining the through-bores 202, 204 in the augments may be smooth (i.e., non-threaded) and the through-bores 204 for the screws may have top and bottom countersinks so that the augment may be used on either the medial or lateral side, as disclosed in US-7175665. As shown in FIG. 26, when the augment is mounted on the tibial tray 14A, one surface 210 of the augment bears against distal surface 28A of the porous metal portion 82A of the tibial tray 14A and the opposite surface 212 of the augment 200 becomes the bone-engaging surface of this side of the tibial tray 14A.

The augment 200 may comprise a porous metal foam. For example, the augment 200 may be made using processes such as those disclosed in US-A-2008/199720, US-A-2010/098574, US-A-2009/0326674 and US-A-2009/292365. Exposed peripheral surfaces of the augments, such as surface 250 in FIGS. 25 and 26, may be treated to smooth the exposed peripheral surface 250. The smoothing treatment may comprise, for example, machining as discussed above; alternatively or in addition, the surface 250 may be masked during any process used to roughen other surfaces of the augment.

To use the system of the present invention, the surgeon would prepare the distal femur and proximal tibia to receive the bone implants 12, 14, 14A using conventional techniques and implant the tibial tray and femoral component using conventional techniques for cementless components. The tibial bearing 16 is typically assembled with the tibial tray 14, 14A after the tray 14, 14A has been implanted.

After implantation, it is anticipated that bone will grow into the porous metal portion 82, 82A of the tibial tray 14, 14A and porous metal portion 83 of the femoral component 12, including the pegs 32, 34, 36, 38, 39, 32A, 34A, 36A, 38A and stem 30, 30A. If the pegs and stem are made with smoother free ends 40, 42, 44, 46, 48, 51, 40A, 42A, 44A, 46A bone will not, however, grow or grow as vigorously into the smoother free ends. Thus, it is anticipated that there will be bone ingrowth into the distal surface 28, 28A of the tibial platform 24, 24A and porous metal portion 83 of the femoral component 12. In addition, bone ingrowth is also anticipated into the exterior surfaces 70, 72, 76, 79, 70A, 72A, 76A of the extensions 30, 32, 34, 36, 38, 39, 30A, 32A, 34A, 36A, 38A adjacent to the distal surface 28 of the tibial platform 24 and porous metal portion 83 of the femoral component 12 as well as at the junctions 60, 62, 66, 69, 60A, 62A, 66A. Radial pressure along the proximal exterior surfaces 70, 72, 76, 79, 70A, 72A, 76A is expected to be uniform, to stimulate bone ingrowth in all directions on the stem and pegs 30, 32, 34, 36, 38, 39, 30A, 32A, 34A, 36A, 38A. If the free ends 40, 42, 44, 46, 48, 51, 40A, 42A, 44A, 46A of the pegs and stem are smoother (or comprise solid material) than the rest of the porous metal portion, bone is not expected to grow or to grow as vigorously into the smoother exposed exterior surfaces at the free ends 40, 42, 44, 46, 48, 51, 40A, 42A, 44A, 46A of the extensions 30, 32, 34, 36, 38, 39, 30A, 32A, 34A, 36A, 38A.

The extensions 30, 32, 34, 36, 38, 39, 30A, 32A, 34A, 36A, 38A stabilize the implant component 12, 14, 14A when implanted in a bone of a patient. The central stem 30, 30A provides stability against lift off for the tibial tray. The pegs 32, 34, 36, 38, 32A, 34A, 36A, 38A surrounding the central stem 30, 30A and pegs 39 of the femoral component 12 provide stability by reducing shear and micromotion, especially after bone ingrowth has occurred.

If the exposed peripheral surfaces 150, 250 of the implant components are smooth, no soft tissue irritation should occur after the components are implanted and blood should not flow through the porous metal portion into the joint space.

If it later becomes necessary to remove the tibial tray 14, 14A or femoral component 12, the surgeon may cut along the distal bone-engaging surface 28, 28A of the tibial tray platform 24, 24A (or along the distal surface 212 of an augment 200) to sever the connection between the patient's bone and the tibial tray platform 24, 24A at the interface. If the pegs 32, 34, 36, 38, 39, 32A, 34A, 36A, 38A and stem 30, 30A consist of porous metal foam across their entire thicknesses T1 and T2, the surgeon may also cut through all of the extensions 30, 32, 34, 36, 38, 39, 30A, 32A, 34A, 36A, 38A at the junctures 60, 62, 66, 69, 60A, 62A, 66A of the extensions 30, 32, 34, 36, 38, 39, 30A, 32A, 34A, 36A, 38A and the distal surface 28, 28A of the tibial platform 24, 24A and bone-engaging surfaces 13, 15 of the femoral component 12 using a bone saw and easily remove the tibial platform 24, 24A and femoral component 12. Such a result is generally not possible with pegs and stems made of solid titanium or cobalt chromium alloy, since bone saws cannot generally cut through solid metal. To remove the extensions 30, 32, 34, 36, 38, 39, 30A, 32A, 34A, 36A, 38A, the surgeon may then cut around the outer perimeter of each extension 30, 32, 34, 36, 38, 39, 30A, 32A, 34A, 36A, 38A to sever the connection between the bone and the extensions 30, 32, 34, 36, 38, 39, 30A, 32A, 34A, 36A, 38A. Such cuts around the perimeters may be made, for example, through use of a trephine saw. Each extension 30, 32, 34, 36, 38, 39, 30A, 32A, 34A, 36A, 38A may then be readily removed. Notably, if the free ends of the extensions are smooth, little or no bone ingrowth will have occurred at the ends of the extensions, so the removal of the stem and pegs should be made easier.

As indicated above, sawing through the stem and pegs 30, 32, 34, 36, 38, 39, 30A, 32A, 34A, 36A, 38A, 30D, 32D, 36D, 30E, 32E, 36E is made easier if the stem and pegs at the junctions 60, 62, 66, 69, 60A, 62A, 66A, 60D, 62D, 66D, 60E, 62E, 66E consist of porous metal rather than solid metal. Generally, it is believed that the stem and pegs may be cut through transversely with a standard surgical saw if the material is 25 to 35% of theoretical density. Notably, in the illustrated embodiments, the titanium alloy studs 132, 134, 136, 138, 140, 132A, 134A, 136A, 138A, 140A, 134D, 134E do not extend beyond the plane of bone-engaging surface 28, 28A, 28D, 28E; therefore, in cutting through the extensions 30, 32, 34, 36, 38, 39, 30A, 32A, 34A, 36A, 38A, 30D, 32D, 36D, 30E, 32E, 36E, the surgeon need not cut through the solid metal studs 132, 134, 136, 138, 140, 132A, 134A, 136A, 138A, 140A, 134D, 134E.

It is anticipated that a standard surgical saw could cut through a somewhat more dense material. In addition, it is anticipated that a standard surgical saw could cut through a composite of materials, such as a small diameter central core of solid metal (e.g. titanium alloy) surrounded by a porous metal foam (e.g. commercially pure titanium). Accordingly, although for purposes of ease of removal, it is preferred that the entire thicknesses of the extensions be porous metal at the junctions, other considerations may call for a composite of materials to be used.

Thus, the present invention provides a knee prosthesis with a tibial implant component and femoral component suitable for optimized cementless fixation. Soft tissue irritation and bleed-through may be substantially reduced or eliminated. The strength of a sintered bond between porous and solid metal portions of an implant component may be optimized. Moreover, the implant components may be readily removed from the bone in revision surgery to conserve native bone.

The number and configurations of the extensions may be varied. For a tibial tray, for example, the tray could include pegs but no central stem. Although the illustrated tibial trays have four pegs, fewer pegs may be acceptable.

Other variations are possible as well. For example, the extensions 30, 32, 34, 36, 38, 39, 30A, 32A, 34A, 36A, 38A, 30D, 32D, 36D, 30E, 32E, 36E could be made as modular components to be assembled with a base plate intraoperatively if desired. The base plate could comprise a porous preform like that shown in FIG. 8 at 85 sintered to a solid metal portion such as that shown at 80 in FIG. 5. The threaded and Morse taper connections described above should be sufficient to hold the components together without sintering, particularly if the studs are longer, as shown in the embodiment of FIGS. 27 to 29. The extensions and base plate may be provided in a kit form, with the base plate and extensions being discrete components as shown in FIGS 8, 9 and 17 to 20; the extensions in the kit could have differing properties, such as size or surface finish, and the surgeon may chose the most appropriate extension for the particular patient intraoperatively. For example, a set of extensions could be provided with porous distal ends and a second set of extensions could be provided with smooth distal ends to accommodate surgeon preference.

## Claims

1. A knee prosthesis comprising:
a femoral component (12) having an articulation surface (18, 20) and an opposite bone-engaging surface (13, 15),
a bearing (16) having an articulation surface (17) shaped to bear against the articulation surface of the femoral component and an opposite surface (19), and
a tibial component (14) having a mounting surface (26) for the bearing and an opposite bone-engaging surface (28) which is planar,
in which the tibial component includes a recess (122, 124, 126, 128, 130) in its bone-engaging surface and a stud (132, 134, 136, 138, 140) in the recess,
the prosthesis including an extension (30, 32, 34, 36, 38) which is received in the recess and mounted on the stud,
**characterised in that** the length of the stud is such that its free end does not extend beyond the plane of the bone-engaging surface of the tibial component, and the extension extends out from the bone-engaging surface to an end (40, 42, 44, 46, 48) and has an outer surface (60, 62, 66) between its end and the bone-engaging surface.

2. The knee prosthesis of claim 1 in which the stud (132, 134, 136, 138, 140) is threaded and in which the extension (30, 32, 34, 36, 38) includes a threaded bore (41, 43, 45, 47, 49) engaging the stud.

3. The knee prosthesis of claim 1 in which the stud (134A) defines a Morse taper post and in which the extension includes a Morse taper bore (41A) engaging the stud.

4. The knee prothesis of claim 1 in which the tibial component (a) includes a solid metal portion (80) in which the recess (122, 124, 126, 128, 130) and the stud (132, 134, 136, 138, 140) are part of the solid metal portion, and preferably includes (b) a porous metal portion (82) in which the bone-engaging surface (28) is part of the porous metal portion.

5. The knee prosthesis of claim 4 in which the porous metal portion (82) and the extension (30, 32, 34, 36, 38) comprise titanium metal foam and the solid metal portion (80) comprises a titanium alloy.

6. The knee prosthesis of claim 5 in which the porous metal portion (82), the solid metal portion (80) and the extension (30, 32, 34, 36, 38) are bonded together through sintering and in which the extension and the porous metal portion meet at a junction.

7. The knee prosthesis of claim 6 in which the junction of the extension (30, 32, 34, 36, 38) and the porous metal portion (82) comprises titanium metal foam.

8. The knee prosthesis of claim 7 in which:
the tibial component (14) includes a solid metal portion (80) in which the recess (122, 124, 126, 128, 130) and the stud (132, 134, 136, 138, 140) are part of the solid metal portion, and includes a porous metal portion (82) in which the bone-engaging surface (28) is part of the porous metal portion,
the knee prosthesis includes a plurality of extensions (30, 32, 34, 36, 38),
the tibial component includes a plurality spaced recesses (122, 124, 126, 128, 130) and a plurality of spaced studs (132, 134, 136, 138, 140), each stud being within one recess and having an end,
each extension is mounted on a stud and bonded to the porous metal portion and solid metal portion through sintering,
each extension meets the porous metal portion at a junction,
the junctions lie in a plane, and
the ends of the studs do not extend beyond the plane of the junctions.

## Patentansprüche

1. Knieprothese umfassend:
eine Femurkomponente (12) mit einer Gelenkfläche (18, 20) und einer gegenüberliegenden Knochenauflagefläche (13, 15),
ein Lager (16) mit einer Gelenkfläche (17), die zum Anliegen an der Gelenkfläche der Femurkomponente geformt ist, und mit einer gegenüberliegenden Fläche (19), und
eine Tibiakomponente (14) mit einer Befestigungsfläche (26) für das Lager und einer gegenüberliegenden Knochenauflagefläche (28), die planar ist,
wobei die Tibiakomponente eine Aussparung (122, 124, 126, 128, 130) in ihrer Knochenauflagefläche und einen Stift (132, 134, 136, 138, 140) in der Aussparung enthält,
wobei die Prothese eine Verlängerung (30, 32, 34, 36, 38) enthält, die in der Aussparung aufgenommen und an dem Stift befestigt ist,
**dadurch gekennzeichnet, dass** die Länge des Stifts derart ist, dass sein freies Ende nicht über die Ebene der Knochenauflagefläche der Tibiakomponente verläuft, und dass die Verlängerung aus der Knochenauflagefläche zu einem Ende (40, 42, 44, 46, 48) verläuft und eine äußere Fläche (60, 62, 66) zwischen ihrem Ende und der Knochenauflagefläche aufweist.

2. Knieprothese nach Anspruch 1, wobei der Stift (132, 134, 136, 138, 140) mit einem Gewinde versehen ist und wobei die Verlängerung (30, 32, 34, 36, 38) eine Gewindebohrung (41, 43, 45, 47, 49), die mit dem Stift in Eingriff steht, enthält.

3. Knieprothese nach Anspruch 1, wobei der Stift (134A) eine Morse Taper-Stange definiert und die Verlängerung eine Morse Taper-Bohrung (41A) enthält, die mit dem Stift in Eingriff steht.

4. Knieprothese nach Anspruch 1, wobei die Tibiakomponente (a) einen festen Metallteil (80) enthält, in dem die Aussparung (122, 124, 126, 128, 130) und der Stift (132, 134, 136, 138, 140) Teil des festen Metallteils sind, und vorzugsweise (b) einen porösen Metallteil (82) enthält, in dem die Knochenauflagefläche (28) Teil des porösen Metallteils ist.

5. Knieprothese nach Anspruch 4, wobei der poröse Metallteil (82) und die Verlängerung (30, 32, 34, 36, 38) Titanmetallschaum umfassen und der feste Metallteil (80) eine Titanlegierung umfasst.

6. Knieprothese nach Anspruch 5, wobei der poröse Metallteil (82), der feste Metallteil (80) und die Verlängerung (30, 32, 34, 36, 38) durch Sintern miteinander verbunden sind und die Verlängerung und der poröse Metallteil sich an einer Verbindung treffen.

7. Knieprothese nach Anspruch 6, wobei die Verbindung der Verlängerung (30, 32, 34, 36, 38) und des porösen Metallteils (82) Titanmetallschaum umfasst.

8. Knieprothese nach Anspruch 7, wobei:
die Tibiakomponente (14) einen festen Metallteil (80) enthält, wobei die Aussparung (122, 124, 126, 128, 130) und der Stift (132, 134, 136, 138, 140) Teil des festen Metallteils sind, und einen porösen Metallteil (82) enthält, wobei die Knochenauflagefläche (28) Teil des porösen Metallteils ist,
die Knieprothese eine Vielzahl von Verlängerungen (30, 32, 34, 36, 38) enthält,
die Tibiakomponente eine Vielzahl von beabstandeten Aussparungen (122, 124, 126, 128, 130) und eine Vielzahl von beabstandeten Stiften (132, 134, 136, 138, 140) enthält, wobei jeder Stift sich in einer Aussparung befindet und ein Ende aufweist,
jede Verlängerung an einem Stift befestigt und mit dem porösen Metallteil und dem festen Metallteil durch Sintern verbunden ist,
jede Verlängerung auf den porösen Metallteil an einer Verbindung trifft,
wobei die Verbindungen in einer Ebene liegen, und
die Enden der Stifte nicht über die Ebene der Verbindungen verlaufen.

## Revendications

1. Prothèse de genou comprenant:
un composant fémoral (12) qui présente une surface d'articulation (18, 20) et une surface de mise en prise d'os opposée (13, 15) ;
une portée (16) qui présente une surface d'articulation (17) dont la forme lui permet de porter contre la surface d'articulation du composant fémoral et une surface opposée (19) ; et
un composant tibial (14) qui présente une surface de montage (26) de la portée et une surface de mise en prise d'os opposée (28) qui est plane ;
dans laquelle le composant tibial comprend un renfoncement (122, 124, 126, 128, 130) dans sa surface de mise en prise d'os et un goujon (132, 134, 136, 138, 140) dans le renfoncement ;
la prothèse comprenant une extension (30, 32, 34, 36, 38) qui est reçue dans le renfoncement et qui est montée sur le goujon ;
**caractérisée en ce que** la longueur du goujon est telle que son extrémité libre ne s'étend pas au-delà du plan de la surface de mise en prise d'os du composant tibial, et l'extension s'étend vers l'extérieur à partir de la surface de mise en prise d'os vers une extrémité (40, 42, 44, 46, 48) et présente une surface extérieure (60, 62, 66) située entre son extrémité et la surface de mise en prise d'os.

2. Prothèse de genou selon la revendication 1, dans laquelle le goujon (132, 134, 136, 138, 140) est fileté, et dans laquelle l'extension (30, 32, 34, 36, 38) comprend un alésage fileté (41, 43, 45, 47, 49) qui vient en prise avec le goujon.

3. Prothèse de genou selon la revendication 1, dans laquelle le goujon (134A) définit une queue cône Morse et dans laquelle l'extension comprend un alésage cône Morse (41A) qui vient en prise avec le goujon.

4. Prothèse de genou selon la revendication 1, dans laquelle le composant tibial comprend : (a) une partie métallique pleine (80) dans laquelle le renfoncement (122, 124, 126, 128, 130) et le goujon (132, 134, 136, 138, 140) font partie de la partie métallique pleine, et comprend de préférence : (b) une partie métallique poreuse (82) dans laquelle la surface de mise en prise d'os (28) fait partie de la partie métallique poreuse.

5. Prothèse de genou selon la revendication 4, dans laquelle la partie métallique poreuse (82) et l'extension (30, 32, 34, 36, 38) comprennent une mousse métallique de titane et la partie métallique pleine (80) comprend un alliage de titane.

6. Prothèse de genou selon la revendication 5, dans laquelle la partie métallique poreuse (82), la partie métallique pleine (80) et l'extension (30, 32, 34, 36, 38) sont collées ensemble par frittage et dans laquelle l'extension et la partie métallique poreuse se rencontrent au niveau d'une jonction.

7. Prothèse de genou selon la revendication 6, dans laquelle la jonction de l'extension (30, 32, 34, 36, 38) et de la partie métallique poreuse (82) comprend une mousse métallique de titane.

8. Prothèse de genou selon la revendication 7, dans laquelle :
le composant tibial (14) comprend une partie métallique pleine (80) dans laquelle le renfoncement (122, 124, 126, 128, 130) et le goujon (132, 134, 136, 138, 140) font partie de la partie métallique pleine, et il comprend une partie métallique poreuse (82) dans laquelle la surface de mise en prise d'os (28) fait partie de la partie métallique poreuse ;
la prothèse de genou comprend une pluralité d'extensions (30, 32, 34, 36, 38) ;
le composant tibial comprend une pluralité de renfoncements espacés (122, 124, 126, 128, 130) et une pluralité de goujons espacés (132, 134, 136, 138, 140), chaque goujon se situant à l'intérieur d'un renfoncement et présentant une extrémité ;
chaque extension est montée sur un goujon et collée sur la partie métallique poreuse et sur la partie métallique pleine par frittage ;
chaque extension rencontre la partie métallique poreuse au niveau d'une jonction ;
les jonctions se situent dans un plan ; et
les extrémités des goujons ne s'étendent pas au-delà du plan des jonctions.
